# EUROPEAN PATENT SPECIFICATION

(11) **EP 2 070 971 B1**
(45) Date of publication and mention of the grant of the patent: **22.06.2016**
(21) Application number: 07828587.1
(22) Date of filing: 27.09.2007
(51) Int. Cl.: C08G 85/00, A61K 31/787, A61P 35/00, C08G 81/00

(54) **COMPOUND OF RESORCINOL DERIVATIVE WITH POLYMER**
RESORCINOLDERIVATVERBINDUNG MIT POLYMER
MÉLANGE D'UN DÉRIVÉ DE RÉSORCINOL AVEC UN POLYMÈRE

(30) Priority: 03.10.2006 JP 2006271425
(43) Date of publication of application: 17.06.2009
(73) Proprietor: Nippon Kayaku Kabushiki Kaisha, Tokyo 102-8172 (JP)
(72) Inventor: NAKAMURA, Masaharu, Tokyo 115-8588 (JP); KITAGAWA, Masayuki, Tokyo 115-8588 (JP); YAMAMOTO, Keiichirou, Tokyo 115-8588 (JP); SENO, Chieko, Tokyo 115-8588 (JP)
(74) Representative: Wablat, Wolfgang
(86) International application number: PCT/JP2007/068841
(87) International publication number: WO 2008/041610

(56) References cited:
- EP-A1- 1 489 125
- WO-A1-2004/039869
- WO-A1-2005/000300
- WO-A1-2005/018674
- WO-A1-2006/055760
- WO-A1-2006/095783
- JP-A- 05 000 955
- JP-A- 10 513 187
- JP-A- 2000 517 304
- JP-A- 2003 527 443
- JP-A- 2005 517 675
- JP-A- 2006 510 627
- JP-A- 2006 511 571
- JP-A- 2006 517 572
- JP-A- 2006 524 673
- JP-B2- 2 894 923
- JP-B2- 3 268 913
- JP-B2- 3 310 000
- LEN NECKERS: 'Hsn90 inhibitors as nobel cancer chemotherapeutic agents' TRENDS IN MOLECULAR MEDICINE vol. 8, no. 4, SUPPL. 1, 2002, pages 55 - 61, XP002246588
- Hermann Hauer ET AL: "An Improved and Large Scale Synthesis of the Natural Coumarin Scopoletin", Archiv der Pharmazie, vol. 328, no. 10, 1 January 1995 (1995-01-01), pages 737-738, XP055183154, ISSN: 0365-6233, DOI: 10.1002/ardp.19953281009

## Description

### Technical Field

The present invention relates to a high molecular weight conjugate of resorcinol derivatives, in which a carboxyl group in a copolymer having a polyethylene glycol moiety and a polymer moiety based on polyglutamic acid having a carboxyl group in the side chain is linked to a hydroxyl group of a resorcinol derivative via an ester bond, a manufacturing method thereof, and an anticancer agent comprising it.

### Background Art

Resorcinol derivatives are known to exhibit anti-tumor activity by binding to proteins of the heat shock protein 90 (HSP90) family and inhibiting the functions of the proteins of the HSP90 family (Non-Patent Document 1). As resorcinol derivatives, compounds having a pyrazole skeleton (Patent Documents 1 to 4), an isoxazole skeleton (Patent Document 5) or a triazole skeleton (Patent Documents 6 to 8) and the like are known. In addition to the resorcinol derivatives, known as a compound which binds to the proteins of the HSP90 family are compounds derived from natural products, such as Geldanamycin, Radicicol (Patent Document 9), and 17-AAG, which is a derivative of Geldanamycin (Non-Patent Document 2); low molecular weight compounds such as PU3, which is a purine derivative, and derivatives thereof; and the like (Non-Patent Document 3). However, many of these compounds are not satisfactory to be used as pharmaceutical products from the aspect of the anti-tumor effect, and even from the aspect of physical properties, many of the compounds have poor water-solubility. For example, 17-AAG, which is currently under a clinical trial, is being administered with a large amount of DMSO. Also, the compounds, which are derived from natural products, have large molecular weights, and often have problems in the stability in the body.

In the meantime, a molecule is known which exhibit water-solubility by linking a drug to a block copolymer of polyethylene glycol and polyaspartic acid to form micelles (Patent Document 10), and a polymer carrier is known which serves as a polymeric drug vehicle having a hydrophobic substance linked to a side chain carboxylic acid of a block copolymer of a polyethylene glycol and a poly (acidic amino acid) (Patent Documents 11 and 12). In Patent Document 12, a discrepancy in anti-tumor effect between the case where the poly (acidic amino acid) is polyglutamic acid, and the case where the poly (acidic amino acid) is polyaspartic acid is described. Thus, it is understood that appropriate selection of the combination of the polymer carrier and the included compound is essential in manifesting the effect. Furthermore, there is also known a high molecular weight conjugate of a camptothecin, in which a side chain carboxyl group of a block copolymer of a polyethylene glycol and polyglutamic acid is linked to a phenolic hydroxyl group of the camptothecin (Patent Document 13). However, Patent Documents 9 to 12 do not provide any description on high-molecular weight conjugates of resorcinol derivatives. Patent Document 14 refers to a high-molecular weight conjugate of a polymer-multicarboxyl oligopeptide and a drug molecule, which is, inter alia, a scopoletin derivative.
Patent Document 1: WO 03/055860
Patent Document 2: WO 04/050087
Patent Document 3: WO 04/056782
Patent Document 4: WO 04/096212
Patent Document 5: WO 04/072051
Patent Document 6: WO 05/000300
Patent Document 7: WO 06/055760
Patent Document 8: WO 05/018674
Patent Document 9: WO 06/095783
Patent Document 10: Japanese Patent No. 2694923
Patent Document 11: Japanese Patent No. 3268913
Patent Document 12: Japanese Patent No. 3310000
Patent Document 13: WO 04/039869
Patent Document 14: EP 1 489 125 A1
Non-Patent Document 1: Hsp90 inhibitors as novel cancer chemotherapeutic agents. Trends Mol Med. 2002; 8(4 Suppl.): p.S55-61.
Non-Patent Document 2: The clinical applications of heat shock protein inhibitors in cancer - present and future. Curr. Cancer Drug Targets. 2003 Oct; 3(5): p.385-390.
Non-Patent Document 3: Vilenchik, M. et al. Targeting wide-range oncogenic transformation via PU24FCl, A specific inhibitor of tumor Hsp90. Chem. Biol. 11, 787-797 (2004).

### Disclosure of the Invention

### Problems to be Solved by the Invention

As described above, inhibitors of the HSP90 family are known to have anti-tumor activity and the like. Although some compounds are under clinical development, compounds having water-solubility and stability, which are properties required as medicine, and sufficiently exhibiting anti-tumor activity, have not been obtained. Thus, HSP90 inhibitors which can be used clinically have been demanded.

### Means for Solving the Problems

To solve the above-mentioned problems, the inventors of the present invention devotedly carried out investigation, and as a result, found a high-molecular weight conjugate of resorcinol derivatives, wherein a hydroxyl group of the resorcinol derivatives is linked to a carboxyl group of a copolymer of a polyethylene glycol moiety and a polymer moiety based on polyglutamic acid having a carboxylic acid group in the side chain via an ester bond, and thus completed the present invention.

Specifically, the present invention relates to the following items (1) to (7).
(1) A high-molecular weight conjugate of resorcinol derivatives, which is a compound represented by the general formula (1): 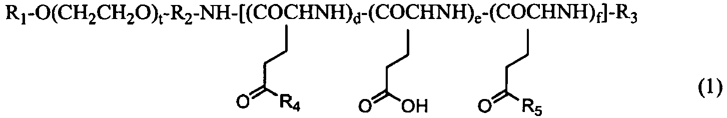 wherein R₁ represents a hydrogen atom, or an alkyl group having 1 to 6 carbon atoms which may have a substituent; R₂ represents a linking group; R₃ represents a hydrogen atom, an acyl group having 1 to 6 carbon atoms, or an alkoxycarbonyl group having 1 to 6 carbon atoms; R₄ represents the residue of the hydroxyl group of the resorcinol derivative; R₅ represents a group selected from the group consisting of an alkoxy group having 1 to 30 carbon atoms, an aralkyloxy group having 7 to 30 carbon atoms, an alkylamino group having 1 to 30 carbon atoms which may have a substituent, a di(C₁-C₃₀) alkylamino group which may have a substituent, an amino acid with a protected carboxyl group, and -N(R₆)CONH(R₇) wherein R₆ and R₇, which may be identical or different, each represent a cyclic alkyl group having 3 to 6 carbon atoms, or an alkyl group having 1 to 5 carbon atoms which may be substituted with a tertiary amino group; t represents an integer from 5 to 11500; d represents an integer from 1 to 200, e and f each represent an integer from 0 to 200, and d+e+f represents an integer from 3 to 200; and the respective constituent units of the polyglutamic acid are bound in any order, wherein the resorcinol derivatives are resorcinol derivatives represented by the general formula (2): 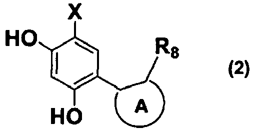 wherein ring A represents a heteroaromatic ring consisting of five atoms, which may have a substituent; X represents a mercapto group, a hydroxyl group, a halogen atom, a nitro group, a cyano group, or an alkyl group, alkenyl group or alkynyl group which may have a substituent, a carbocyclic or heterocyclic aryl group which may have a substituent, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group or carbamoyloxy group, or an amino group, an acylamino group, an alkoxycarbonylamino group, a ureido group, a sulfonylamino group, a sulfamoylamino group, a formyl group, an acyl group, a carboxyl group, an alkoxycarbonyl group, a carbamoyl group and a silyl group which may have a substituent; and R₈ represents a carbocyclic or heterocyclic aryl group which may have a substituent, an alkyl group, alkenyl group or alkynyl group which may have a substituent, or an amino group or acylamino group which may have a substituent,
   wherein the residues of the resorcinol derivatives are linked by a hydroxyl group to a carboxyl group in the side chain of the copolymer via an ester bond.
(2) The high-molecular weight conjugate of resorcinol derivatives according to (1) above, wherein R₁ is an alkyl group having 1 to 6 carbon atoms which may have a substituent; R₂ is an alkylene group having 2 to 6 carbon atoms; R₃ is an acyl group having 1 to 6 carbon atoms, or an alkoxycarbonyl group having 1 to 6 carbon atoms; t is an integer from 100 to 300; d is an integer from 1 to 100, e and f are each an integer from 0 to 100, and d+e+f is an integer from 6 to 100; and R₅ is a group selected from the group consisting of an amino acid with a protected carboxyl group, and -N(R₆)CONH(R₇).
(3) The high-molecular weight conjugate of resorcinol derivatives according to (2) above, wherein R₁ is a methyl group, R₂ is a trimethylene group, R₃ is an acetyl group, and R₅ is an isopropylaminocarbonylisopropylamino group.
(4) The high-molecular weight conjugate of resorcinol derivatives according to any one of (1) to (3) above, wherein the ring A defined in (1) above is any one of the substituents selected from groups of the following formulas (3-1) to (3-7): 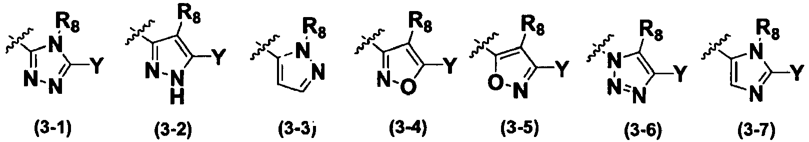 wherein R₈ represents the substituent as defined in (8) above; and Y represents a mercapto group, a hydroxyl group, a hydrogen atom, a halogen atom, a carbamoyl group, an alkoxycarbonyl group, a cyano group, an alkylthio group, an arylthio group, an alkylsulfinyl group, an arylsulfinyl group, an alkylsulfonyl group, an arylsulfonyl group, a sulfamoyl group, an alkoxyl group, an aryloxy group, an acyloxy group, an alkoxycarbonyloxy group, a carbamoyloxy group, or an amino group, an acylamino group, an alkoxycarbonylamino group, a ureido group, a sulfonylamino group, a sulfamoylamino group, a formyl group, an acyl group or a silyl group which may have a substituent.
(5) The high molecular weight conjugate of resorcinol derivatives according to any one of (1) to (4) above, wherein the resorcinol derivatives are selected from the group consisting of groups of formulas (5-1) to (5-21): 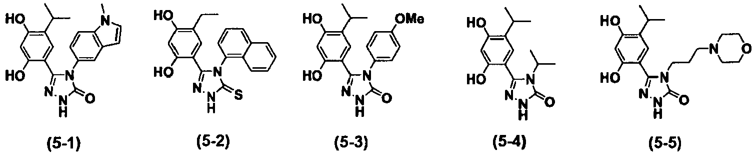 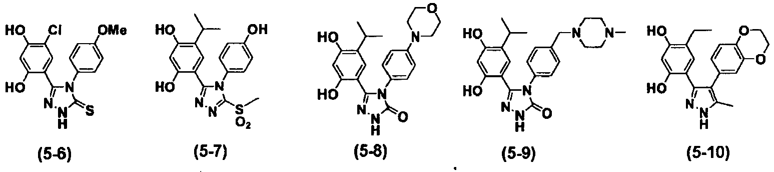 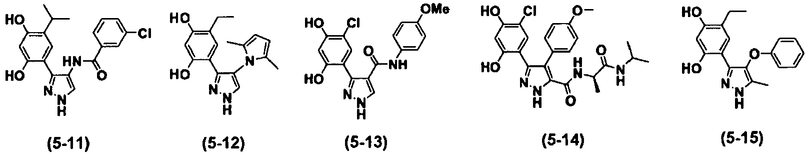 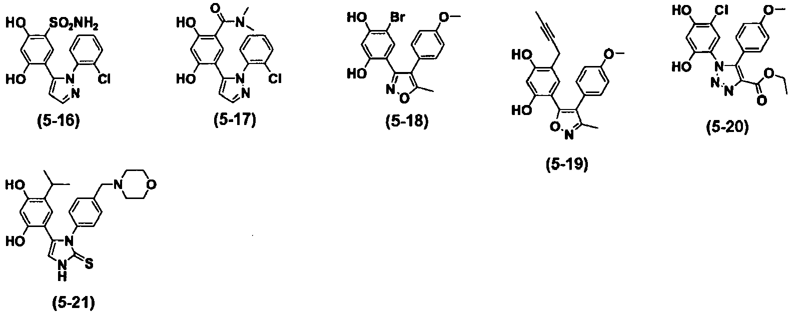
(6) A method for manufacturing a high molecular weight conjugate of resorcinol derivatives according to any one of (1) to (5) above, the method comprising linking a carboxyl group of the polymer moiety having a carboxyl group in the side chain and the polyethylene glycol moiety, to a hydroxyl group of the resorcinol derivatives via an ester bond in an organic solvent, using a dehydrating condensing agent.
(7) An anticancer agent comprising, as an active ingredient, the high-molecular weight conjugate of resorcinol derivatives according to any one of (1) to (6) above.

### Effects of the Invention

The present invention provides a high-molecular weight conjugate of resorcinol derivatives, which exhibits anti-tumor activity by inhibiting the HSP90 family, and is excellent in water-solubility and pharmacokinetics. The pharmacokinetics in the body, the stability and the water-solubility of the high-molecular weight conjugate of the present invention has been improved as compared with simple substance of resorcinol derivatives, because of the appropriate combination of a copolymer and resorcinol derivatives in the high-molecular weight conjugate. For this reason, the anti-tumor effect is sustained over an extended period of time, and resorcinol derivatives can be administered without using DMSO, which is conventionally required for dissolving the simple substances of resorcinol derivatives (which is the included compound). In the case of the high-molecular weight conjugate of the present invention, the total amount of administration of the included compound is lowered because of the sustained anti-tumor effect over an extended period of time, and therefore reduction of toxicity is also expected. Furthermore, even in the absence of enzymes, the high-molecular weight conjugate of the present invention effects sustained release of the resorcinol derivatives which exhibits anti-tumor activity.

### Best Mode for Carrying Out the Invention

The high-molecular weight conjugate of resorcinol derivatives of the present invention comprises a structure in which a hydroxyl group of the resorcinol derivatives is linked to a carboxyl group of a copolymer of a polyethylene glycol moiety and a polymer moiety having a carboxyl group in the side chain via an ester bond.

In the present invention, polymer moieties having carboxyl groups in the side chain are polyglutamic acid.

According to the present invention, examples of polyethylene glycol moiety include polyethylene glycols modified at both ends or at one end, and in the case where the both ends are modified, the modifying groups may be identical or different. Examples of the terminal modifying group include an alkyl group having 1 to 6 carbon atoms which may have a substituent. Specific examples thereof include a methyl group, an ethyl group, an n-propyl group, an i-propyl group, an n-butyl group, an s-butyl group, a t-butyl group, a benzyl group, a dimethoxyethyl group, a diethoxyethyl group, and the like. Preferred is an alkyl group having 1 to 4 carbon atoms which may have a substituent, and specific examples thereof include a methyl group, an ethyl group, an n-propyl group, an i-propyl group, an n-butyl group, an s-butyl group, a t-butyl group, a dimethoxyethyl group and the like. Preferred is an alkoxy polyethylene glycol, and more preferred is methoxy polyethylene glycol.

The molecular weight of the polyethylene glycol moiety is generally about 300 to 500,000, preferably about 500 to 100,000, and more preferably about 1000 to 50,000.

The average number of carboxyl groups per molecule of the copolymer of a polyethylene glycol moiety and a polymer moiety based on polyglutamic acid having a carboxyl group in the side chain, is about 1 to 300, preferably 3 to 200, and more preferably 6 to 60. The number of carboxyl groups is determined by neutralization titration with alkali.

According to the present invention, the copolymer of a polyethylene glycol moiety and a polymer moiety based on polyglutamic acid having a carboxyl group in the side chain includes graft type polymers and block type polymers, and preferred are block type polymers.

Examples of the copolymer having a polyethylene glycol structural moiety and a polymer moiety based on polyglutamic acid having a carboxyl group in the side chain include alkoxy polyethylene glycol-polyglutamic acid.

The molecular weight of the copolymer having a polyethylene glycol structural moiety and a polymer moiety based polyglutamic acid having a carboxyl group in the side chain according to the present invention is generally about 500 to 500, 000, preferably about 600 to 100,000, and more preferably 800 to 80,000. According to the present invention, the molecular weight as used herein refers to a weight average molecular weight determined by a GPC method.

According to the present invention, the amount of the resorcinol derivatives linked to the copolymer having a polyethylene glycol moiety and a polymer moiety based polyglutamic acid having a carboxyl group in the side chain, is not particularly limited as long as it is an efficacious amount, but the amount of the derivative linked to the copolymer is generally 1 to 100%, preferably 10 to 90%, of the total number of carboxyl groups.

The definitions for the respective groups used in the present invention will be set forth in the following. However, if otherwise specified, exception will be made.

The halogen atom represents a fluorine atom, a chlorine atom, a bromine atom or an iodine atom.

The alkyl group represents, unless otherwise specified, a linear, branched or cyclic alkyl group having 1 to 20 carbon atoms, and preferably 1 to 8 carbon atoms. Examples of the linear alkyl group include a methyl group, an ethyl group, a propyl group, an n-butyl group, an n-pentyl group, an n-hexyl group, and the like. Examples of the branched alkyl group include an isopropyl group, a tert-butyl group, a 2,2-dimethylpropyl group, and the like. Examples of the cyclic alkyl group include a cyclopropyl group, a cyclobutyl group, a cyclopentyl group, a cyclohexyl group, an adamantyl group, and the like.

The alkenyl group has a carbon-carbon double bond at any one or more sites represents, and represents, unless otherwise specified, a linear, branched or cyclic alkenyl group having 2 to 20 carbon atoms, preferably 2 to 8 carbon atoms. Examples of the linear alkenyl group include an ethenyl group; a 1-alkenyl group such as a 1-propenyl group or a 1-butenyl group; a 2-alkenyl group such as a 2-butenyl group or a 2-pentenyl group; and the like. Examples of the branched alkenyl group include an isopropenyl group, a 3-methyl-1-butenyl group, a geranyl group, and the like.

The alkynyl group has a carbon-carbon triple bond at any one or more sites, and represents, unless otherwise specified, an alkynyl group having 2 to 20 carbon atoms, preferably 2 to 8 carbon atoms. Examples thereof include an ethynyl group; a 1-alkynyl group such as a 1-propynyl group or a 3,3-dimethyl-1-butynyl group; a 2-alkynyl group such as a 2-propynyl group, a 2-butynyl group, a 3-phenyl-2-propynyl group, a 4,4-dimethyl-2-pentynyl group, a 3-trimethylsilyl-2-propynyl group; and the like.

Examples of carbocyclic aryl group include a phenyl group, a naphthyl group and the like.

Examples of the heterocyclic aryl group include a pyridyl group, a pyrimidinyl group, a quinolyl group, a quinazolyl group, a naphthyridinyl group, a furyl group, a pyrrolyl group, an indolyl group, an imidazolyl group, a pyrazolyl group, an oxazolyl group, an isoxazolyl group, a triazolyl group, and the like.

Examples of the substituent in the case defined by the phrase "which may have a substituent" include a hydrogen atom, a mercapto group, a hydroxyl group, a halogen atom, a nitro group, a cyano group, an alkyl group, an alkenyl group, an alkynyl group, a carbocyclic or heterocyclic aryl group, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group, a carbamoyloxy group, an amino group, an acylamino group, an alkoxycarbonylamino group, a ureido group, a sulfonylamino group, a sulfamoylamino group, a formyl group, an acyl group, a carboxyl group, an alkoxycarbonyl group, a carbamoyl group, a silyl group, and the like.

The position of substitution on an aromatic ring may be any of the ortho-position, meta-position and para-position.

The alkylthio group represents, unless otherwise specified, an alkylthio group having 1 to 8 carbon atom, and examples thereof include a methylthio group, an isopropylthio group, a benzylthio group and the like. Examples of the arylthio group include a phenylthio group, a naphthylthio group, a pyridylthio group, and the like. The alkylsulfinyl group represents, unless otherwise specified, an alkylsulfinyl group having 1 to 8 carbon atoms, and examples thereof include a methylsulfinyl group, an isopropylsulfinyl group, a benzylsulfinyl group, and the like. Examples of the arylsulfinyl group include a phenylsulfinyl group, a naphthylsulfinyl group, a pyridylsulfinyl group, and the like. Examples of the sulfonyl group which may have a substituent include an alkylsulfonyl group, an alkenylsulfonyl group, an alkynylsulfonyl group, an arysulfonyl group, or the like. The alkylsulfonyl group represents, unless otherwise specified, an alkylsulfonyl group having 1 to 8 carbon atoms, and examples thereof include a methylsulfonyl group, an isopropylsulfonyl group, a benzylsulfonyl group, and the like. Examples of the arylsulfonyl group include a phenylsulfonyl group, a naphthylsulfonyl group, a pyridylsulfonyl group, and the like. Examples of the sulfamoyl group include a dimethylsulfamoyl group, a phenylsulfamoyl group, and the like.

The alkoxy group represents, unless otherwise specified, an alkoxy group having 1 to 8 carbon atoms, and examples thereof include a methoxy group, an isopropoxyl group, a benzyloxy group, and the like. Examples of the aryloxy group include a phenoxyl group, a naphthyloxy group, a pyridyloxy group, and the like. The acyloxy group represents, unless otherwise specified, an acyloxy group having 1 to 8 carbon atoms, and examples thereof include an acetoxyl group, a benzoyloxy group, and the like. The alkoxycarbonyloxy group represents, unless otherwise specified, an alkoxycarbonyloxy group having 1 to 8 carbon atoms, and examples thereof include a methoxycarbonyloxy group, a trifluoromethoxycarbonyl group, and the like. Examples the carbamoyloxy group include a dimethylcarbamoyloxy group, a phenylcarbamoyloxy group, and the like.

Examples of the amino group include an unsubstituted amino group, a dimethylamino group, a morpholino group, a piperidinyl group, a 4-methylpiperazin-1-yl group, a phenylamino group, and the like. Examples of the acylamino group include an acetylamino group, a benzoylamino group, and the like. Examples of the alkoxycarbonylamino group include a methoxycarbonylamino group, an ethoxycarbonylamino group, a benzyloxycarbonylamino group, and the like. Examples of the ureido group include a trimethylureido group, a 1-methyl-3-phenylureido group, and the like. Examples of the sulfonylamino group include a methanesulfonylamino group, a benzenesulfonylamino group, and the like. Examples of the sulfamoylamino group include a dimethylsulfamoylamino group, and the like.

Examples of the acyl group include an acetyl group, a pivaloyl group, a benzoyl group, a pyridinecarbonyl group, and the like. Examples of the alkoxycarbonyl group include a methoxycarbonyl group, a benzyloxycarbonyl group, and the like. Examples of the carbamoyl group include a dimethylcarbamoyl group, a phenylcarbamoyl group, and the like.

Examples of the silyl group include a trimethylsilyl group, a triisopropylsilyl group, a tert-butyldiphenylsilyl group, and the like.

Preferably, X in the formula (2) is a halogen atom, an alkyl group which may have a substituent, a carbamoyl group, or a sulfamoyl group, and particularly preferred is a chlorine atom, a bromine atom, an ethyl group, or an isopropyl group. The substituent for X in the formula (2) is preferably a hydrogen atom or a hydroxyl group.

Preferably, R₈ in the formula (2) is a phenyl group which may have a substituent, a naphthyl group, a pyrrolyl group, or an indolyl group. The substituent for R₈ in the formula (2) is preferably a hydroxyl group, a halogen atom, an alkyl group, an alkoxyl group, or an amino group which may have a substituent.

Preferably, the ring A in the formula (2) is preferably an imidazolyl group, a pyrazolyl group, a triazolyl group, or an isoxazolyl group. The substituent for the ring A in the formula (2) is preferably a hydrogen atom, a mercapto group, a hydroxyl group, an alkyl group, an alkylsulfonyl group, a carbamoyl group or an alkoxycarbonyl group, and particularly preferred is a hydrogen atom, a mercapto group, a hydroxyl group, or a methyl group.

According to the present invention, the resorcinol derivatives are not particularly limited as long as it has a resorcinol skeleton and has anti-tumor activity or the like. The resorcinol derivatives also include pharmacologically acceptable salts and prodrugs thereof. The resorcinol derivatives are preferably a compound having the structure represented by the formula (2). More preferably, a structure in which the ring A in the formula (2) is represented by, for example, the formulas (3-1) to (3-7), or the like may be included, and tautomers thereof are also included.

The tautomers correspond to the same compound, the respective isomers of which can be rapidly converted to each other, and are in the relationship as represented by the following formulas (4-1) and (4-2), for example.

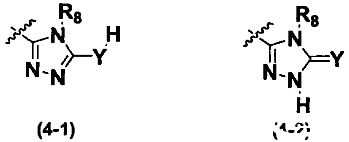

Specific examples of resorcinol derivatives according to the present invention include, but not limited to, structures represented by the following formulas:

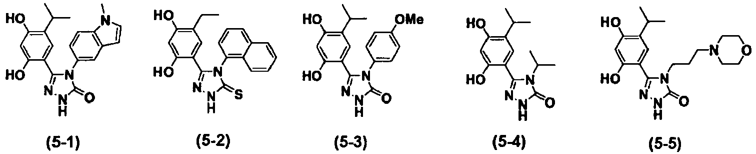

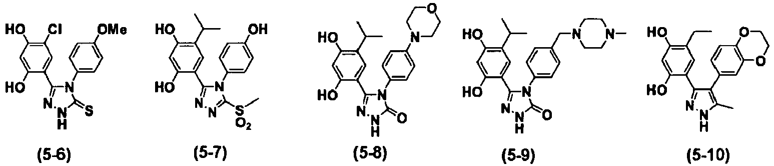

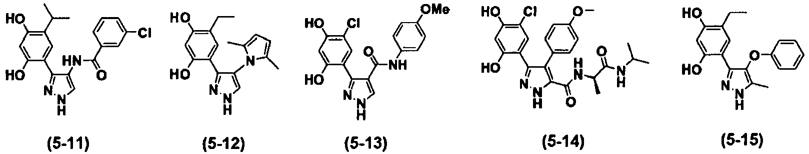

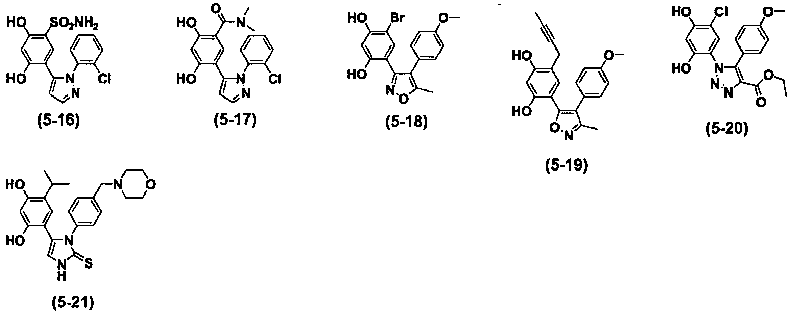

Examples of the high-molecular weight conjugate of resorcinol derivatives of the present invention include a compound represented by the aforementioned formula (1), wherein R₁ represents a hydrogen atom or an alkyl group having 1 to 6 carbon atoms which may have a substituent; R₂ represents a linking group; R₃ represents a hydrogen atom, an acyl group having 1 to 6 carbon atoms, or an alkoxycarbonyl group having 1 to 6 carbon atoms; R₄ represents the residue of a hydroxyl group of the resorcinol derivatives; R₅ represents a group selected from the group consisting of an alkoxy group having 1 to 30 carbon atoms which may have a substituent, an aralkyloxy group having 7 to 30 carbon atoms which may have a substituent, an alkylamino group having 1 to 30 carbon atoms which may have a substituent, an alkylamino group having 1 to 30 carbon atoms which may have a substituent, an amino acid with a protected carboxyl group, and -N(R₆)CONH(R₇) wherein R₆ and R₇, which may be identical or different, each represent a cyclic alkyl group having 3 to 6 carbon atoms, or an alkyl group having 1 to 5 carbon atoms which may be substituted with a tertiary amino group; t represents an integer from 5 to 11500; d represents an integer from 1 to 200; e and f each represent an integer from 0 to 200; and d+e+f represents an integer from 3 to 200.

The alkyl group having 1 to 6 carbon atoms for R₁ in the formula (1) may be exemplified by a straight-chained or branched alkyl group having 1 to 6 carbon atoms which may have a substituent, and examples thereof include a methyl group, an ethyl group, an n-propyl group, an i-propyl group, an n-butyl group, a t-butyl group, and the like. A straight-chained or branched alkyl group having 1 to 4 carbon atoms is preferred, and particularly preferred is a straight-chained or branched alkyl group having 1 to 3 carbon atoms, for example, a methyl group, an ethyl group, an n-propyl group, or an i-propyl group, and a methyl group is more preferred. An unsubstituted alkyl group or an alkyl group having an alkyl group as the substituent is preferred.

Examples of the linking group represented by R₂ in the formula (1) include, but not particularly limited to, an alkylene group having 2 to 6 carbon atoms. An alkylene group having 2 to 4 carbon atoms is preferred, and examples thereof include an ethylene group, a trimethylene group, a butylene group and the like, and particularly preferred is a trimethylene group.

The acyl group having 1 to 6 carbon atoms for R₃ in the formula (1) is not particularly limited, and examples thereof include a formyl group, an acetyl group, a propionyl group, a pivaloyl group, and the like. An acyl group having 1 to 3 carbon atoms is preferred, and an acetyl group is particularly preferred.

The alkoxycarbonyl group having 1 to 6 carbon atoms for R₃ in the formula (1) is not particularly limited, and examples thereof include a methoxycarbonyl group, an ethoxycarbonyl group, and a t-butoxycarbonyl group.

In relation to the residue of resorcinol derivatives, which is R₄ in the formula (1), example of resorcinol derivatives include the aforementioned resorcinol derivatives, and the resorcinol derivatives are not particularly limited as long as they have a hydroxyl group capable of linking to a carboxylic acid moiety of the polymer via an ester bond using a dehydrating condensing agent and have anti-tumor activity.

R₅ in the formula (1) represents a group selected from the group consisting of an alkoxy group having 1 to 30 carbon atoms, an aralkyloxy group having 7 to 30 carbon atoms, an alkylamino group having 1 to 30 carbon atoms which may have a substituent, a di(C₁-C₃₀) alkylamino group which may have a substituent, an amino acid with a protected carboxyl group, and -N(R₆)CONH(R₇), wherein R₆ and R₇, which may be identical or different, each represent a cyclic alkyl group having 3 to 6 carbon atoms, or an alkyl group having 1 to 5 carbon atoms which may be substituted with a tertiary amino group. R₅ in the formula (1) may be identical or different in one molecule, and the polymer used for the high-molecular weight conjugate of resorcinol derivatives may be a single substance, or may also be a mixture.

The alkoxy group having 1 to 30 carbon atoms for R₅ in the formula (1) may be exemplified by a straight-chained or branched alkoxy group having 1 to 30 carbon atoms, and preferred is a straight-chained or branched alkoxy group having 1 to 10 carbon atoms, and examples thereof include a methoxy group, an ethoxy group, an n-propoxy group, an i-propoxy group, an n-butoxy group, a t-butoxy group, and the like. Example of aralkyloxy group having 7 to 30 carbon atoms include a straight-chained or branched aralkyloxy group having 7 to 30 carbon atoms, and preferred is a straight-chained or branched aralkyloxy group having 7 to 20 carbon atoms, and examples thereof include a 4-phenylbutoxy group and the like.

For R₅ in the formula (1), the alkylamino group having 1 to 30 carbon atoms which may have a substituent or the di (C₁-C₃₀) alkylamino group which may have a substituent may be exemplified by a straight-chained or branched alkylamino group having 1 to 30 carbon atoms or a di(C₁-C₃₀) alkylamino group. Preferred is a straight-chained or branched alkylamino group having 1 to 20 carbon atoms or a di(C₁-C₂₀) alkylamino group, and examples thereof include a methylamino group, an ethylamino group, an n-propylamino group, an i-propylamino group, an n-butylamino group, a t-butylamino group, a benzylamino group, an acetylamino group, a dimethylamino group, a diethylamino group, a dipropylamino group, a diisopropylamino group, a dibutylamino group, a dibenzylamino group, a methylbenzylamino group, and the like. An unsubstituted alkylamino group or an alkylamino group having an alkyl group as the substituent is preferred.

For R₅ in the formula (1), the amino acid with a protected carboxyl group may be exemplified by an amino acid used in conventional peptide synthesis, in which a carboxyl group is protected, and examples thereof include phenylalanine benzyl ester, and the like.

For R₅ in the formula (1), the group -N(R₆)CONH(R₇), wherein R₆ and R₇, which may be identical or different, are each a cyclic alkyl group having 3 to 6 carbon atoms, or an alkyl group having 1 to 5 carbon atoms which may be substituted with a tertiary amino group, is not particularly limited, and examples thereof include a cyclohexylaminocarbonylcyclohexylamino group, an isopropylaminocarbonylisopropylamino group, and the like.

The total number of glutamic acid in the high-molecular weight conjugate of resorcinol derivatives represented by the formula (1) is represented by d+e+f, and the number is about 3 to 200, preferably about 6 to 100, and more preferably about 6 to 40.

The proportion of the number of glutamic acid linked to the resorcinol derivatives, d, to the total number of glutamic acid (d+e+f), is 1 to 100%, preferably 10 to 90%.

In the formula (1), t is an integer from about 5 to 11500, but is preferably an integer from about 8 to 2300, and more preferably an integer from about 100 to 300.

The high-molecular weight conjugate of resorcinol derivatives represented by the formula (1) may form micelles in water, with the polyethylene glycol moiety forming the outer shell of the micelle.

The high-molecular weight conjugate of resorcinol derivatives of the present invention may be obtained by linking a carboxyl group of a polymer moiety based on polyglutamic acid having a carboxyl group in the side chain and a polyethylene glycol moiety, to a hydroxyl group of the resorcinol derivatives via an ester bond, in an organic solvent using a dehydrating condensing agent, and this manufacturing method is also included in the present invention. That is, it is a manufacturing method in which, for example, a block copolymer of a polyethylene glycol moiety-polyglutamic acid prepared according to the method described in Patent Document 11, and resorcinol derivatives in which functional groups other than the groups to be reacted are protected as necessary, are subjected to a reaction in a solvent, preferably in an aprotic polar solvent such as N,N-dimethylformamide (DMF), 1,3-dimethyl-2-imidazolidinone (DMI) or N-methylpyrrolidone (NMP), at 0 to 180°C, and preferably at 5 to 50°C, using a dehydrating condensing agent such as dicyclohexylcarbodiimide (DCC), diisopropylcarbodiimide (DIPC), 1-ethyl-3-(3-dimethylaminopropyl)carbodiimide hydrochloride (WSC) or 1-ethoxycarbonyl-2-ethoxy-1,2-dihydroxyquinolinone (EEDQ). In addition, during the condensation reaction, a reaction aid such as N,N-dimethylaminopyridine (DMAP) may also be used. After the condensation reaction, deprotection is carried out as necessary, and conventional operations such as separation and purification are carried out to produce the high-molecular weight conjugate of resorcinol derivatives.

Furthermore, a high-molecular weight conjugate of resorcinol derivatives in which R₅ is the group -N(R₆)CONH(R₇), wherein R₆ and R₇, which may be identical or different, are each a cyclic alkyl group having 3 to 6 carbon atoms or an alkyl group having 1 to 5 carbon atoms which may be substituted with a tertiary amino group, may also be obtained by a reaction using the aforementioned carbodiimides as a condensing agent.

As the method for introducing an alkoxy group having 1 to 30 carbon atoms, an aralkyloxy group having 7 to 30 carbon atoms, an alkylamino group having 1 to 30 carbon atoms, a di (C₁-C₃₀) alkylamino group or an amino acid having the carboxyl group protected, to R₅ in the compound of formula (1), there may be mentioned a method in which the carboxyl group of the polymer is first activated by the method as described above, and then reacted with an amount desired to be linked of a corresponding alcohol, a corresponding amine, an amino acid with a protected carboxyl group or the like under basic conditions; a method in which a corresponding alcohol, a corresponding amine, an amino acid with a protected carboxyl group, or the like is first activated, and then subjected to the condensation reaction with the polymer; or the like. After purification of the polymer, it is possible to re-activate unreacted carboxylic acid groups in the polymer by the same reaction, and hydroxyl groups of resorcinol derivatives may be condensed with the re-activated carboxylic acid groups. Alternatively, different alcohols, amines and the like may b e repeatedly reacted to synthesize a mixture of polymers in which R5 is substituted with various substituents, to which hydroxyl groups of the resorcinol derivatives may subsequently be condensed therewith. Further, after condensation of the resorcinol derivatives, an alkoxy group having 1 to 30 carbon atoms, an aralkyloxy group having 7 to 30 carbon atoms, an alkylamino group having 1 to 30 carbon atoms, a di(C₁-C₃₀) alkylamino group, an amino acid with a protected carboxyl group or the like may be introduced. However, the method for manufacturing the high-molecular weight conjugate of resorcinol derivatives of the present invention is not intended to be limited to the aforementioned methods.

The present invention also includes an anticancer agent comprising the high-molecular weight conjugate of resorcinol derivatives of the present invention as an active ingredient. The high-molecular weight conjugate can be used in a dosage form which is conventionally used, including, for example, injections, tablets, and powders. Pharmaceutically acceptable carriers which are conventionally used in formulation processes, for example, binding agents, lubricants, disintegrants, solvents, excipients, solubilizing agents, a dispersant, stabilizers, suspending agents, preservatives, soothing agents, colorants, flavors and the like can be used. It is preferrd to use the anticancer agent in the form of injection, and typically, for example, water, physiological saline, a 5% glucose or mannitol solution, water-soluble organic solvents (for example, glycerol, ethanol, dimethylsulfoxide, N-methylpyrrolidone, polyethylene glycol, Cremophor or the like, or a mixed liquid thereof), or a mixed liquid of water and water-soluble organic solvents and the like are used.

The dosage of the high-molecular weight conjugate of resorcinol derivatives of the present invention may vary, as a matter of course, with the sex, age and physiological condition, the pathological condition and the like of the patient, but the high-molecular weight conjugate is usually administered parenterally at a dose of 0.01 to 500 mg/m², preferably 0.1 to 250 mg/m² as the active ingredient per day for an adult. Administration by injection is carried out intravenously, intra-arterially, in the affected sites (tumor sites) and the like.

### EXAMPLES

Hereinafter, the present invention will be described more specifically by way of Examples, but the present invention is not intended to be limited to these Examples.
Example 1 Production of compound 1 (conjugate of 5-(2,4-dihydroxy-5-isopropyl-phenyl)-4-(4-methoxy-phenyl)-2 ,4-dihydro-[1,2,4]triazol-3-one (formula (5-3)) and a block copolymer comprising a methoxy polyethylene glycol moiety having a molecular weight of 12000 and a polyglutamic acid moiety having a polymerization number of about 23: in formula (1), R₁ = Me (methyl group), R₂ = trimethylene group, R₃ = Ac (acetyl group), R₄ = hydroxyl group of resorcinol derivatives, R₅ = isopropylaminocarbonylisopropylamino group, d+e+f = 23, t = 273)
   A block copolymer of methoxy polyethylene glycol-polyglutamic acid (polymerization number of glutamic acid: about 23; 1.10 g) prepared according to the method described in Patent Document 12 (Reference Example 1) was dissolved in dimethylformamide (47 ml). After the solution was stirred at room temperature for a while, 5-(2,4-dihydroxy-5-isopropyl-phenyl)-4-(4-methoxy-phenyl)-2,4-dihydro-[1,2,4]t riazol-3-one (260 mg) synthesized according the method described in Patent Document 8, dimethylaminopyridine (28 mg) and diisopropylcarbodiimide (0.47 ml) were added, and the mixture was further stirred for 20 hours at 26°C. After completion of the reaction, ethyl acetate (70 ml), ethanol (70 ml), and diisopropyl ether (564 ml) were added to the reaction liquor. After stirred at room temperature, the reaction mixture was left to stand until the desired product precipitated, and the supernatant was removed. Further, the obtained precipitate was washed twice with ethanol/diisopropyl ether (1/4 (v/v) ; 500 ml), and collected by filtration. The resulting solids were dissolved in acetonitrile/water (9/1 (v/v); 100 ml), and then the solution was passed through a column of ion-exchange resin (Dowex 50 (H+) manufactured by Dow Chemical, Inc.; 10 ml), and eluted with acetonitrile/water (9/1 (v/v); 30 ml). Water (50 ml) was added to the obtained eluted fraction, and then acetonitrile was distilled off under reduced pressure. Then, the residue was freeze-dried to obtain compound 1 (1.04 g). As a result of HPLC (high performance liquid chromatography) measurement of compound 1, free resorcinol derivatives was not detected in compound 1. The content of the resorcinol derivatives in compound 1 can be determined by weighing a portion of compound 1, hydrolyzing the portion under alkaline conditions, and quantifying the resorcinol derivatives cleaved from the methoxy polyethylene glycol-polyglutamic acid block copolymer by HPLC. The content in the present compound 1 was determined by this method, and was found to be 15.1% (w/w).
Example 2 Production of compound 2 (conjugate of 4-{5-hydroxy-4-[4-(morpholin-4-yl)-phenyl]-4H-[1,2,4]triazo 1-3-yl}-6-isopropyl-benzene-1,3-diol (formula (5-8)) and a block copolymer comprising a methoxy polyethylene glycol moiety having a molecular weight of 12000 and a polyglutamic acid moiety having a polymerization number of about 23: in formula (1), R₁ = Me (methyl group), R₂ = trimethylene group, R₃ = Ac (acetyl group), R₄ = hydroxyl group of resorcinol derivatives, R₅ = isopropylaminocarbonylisopropylamino group, d+e+f = 23, t = 273)
   Compound 2 (524 mg) was synthesized according to the same operation as that in Example 1, using 4-{5-hydroxy-4-[4-(morpholin-4-yl)-phenyl]-4H-[1,2,4]triazol-3-yl}-6-isopropyl-benzene-1,3-diol (80 mg) instead of 5-(2,4-dihydroxy-5-isopropyl-phenyl)-4-(4-methoxy-phenyl)-2,4-dihydro-[1,2,4]triazol-3-one of Example 1. Here, the content in compound 2 was 14.57% (w/w).
Example 3 Production of compound 3 (conjugate of 4-(but-2-ynyl)-6-[4-(4-methoxy-phenyl)-5-methyl-isoxazol-3-yl]-benzene-1,3-diol (formula (5-19)) and a block copolymer comprising a methoxy polyethylene glycol moiety having a molecular weight of 12000 and a polyglutamic acid moiety having a polymerization number of about 23: in formula (1), R₁ = Me (methyl group), R₂ = trimethylene group, R₃ = Ac (acetyl group), R₄ = hydroxyl group of resorcinol derivative, R₅ = isopropylaminocarbonylisopropylamino group, d+e+f = 23, t = 273)
   Compound 3 (21.6 mg) was synthesized according to the same operation as that in Example 1, using 4-(but-2-ynyl)-6-[4-(4-methoxy-phenyl)-5-methyl-isoxazol-3-yl]-benzene-1,3-diol (3.4 mg) instead of 5-(2,4-dihydroxy-5-isopropyl-phenyl)-4-(4-methoxy-phenyl)-2,4-dihydro-[1,2,4]triazol-3-one of Example 1. Here, the content in compound 3 was 10.4% (w/w).
Example 4 Production of compound 4 (conjugate of 5-(2,4-dihydroxy-5-isopropyl-phenyl)-4-isopropyl-2,4-dihydro-[1,2,4]triazol-3-one (formula (5-4)) and a block copolymer comprising a methoxy polyethylene glycol moiety having a molecular weight of 12000 and a polyglutamic acid moiety having a polymerization number of about 23: in formula (1), R₁ = Me (methyl group), R₂ = trimethylene group, R₃ = Ac (acetyl group), R₄ = hydroxyl group of resorcinol derivative, R₅ = isopropylaminocarbonylisopropylamino group, d+e+f = 23, t = 273)
   Compound 4 (1.36 g) was synthesized according to the same operation as that in Example 1, using 5-(2,4-dihydroxy-5-isopropyl-phenyl)-4-isopropyl-2,4-dihydro-[1,2,4]triazol-3-one (277.3 mg) instead of 5-(2,4-dihydroxy-5-isopropyl-phenyl)-4-(4-methoxy-phenyl)-2,4-dihydro-[1,2,4]triazol-3-one of Example 1. Here, the content in compound 4 was 11.3% (w/w).

### Test Example 1 Release of the included drug from the compound of the present invention in the absence of enzymes

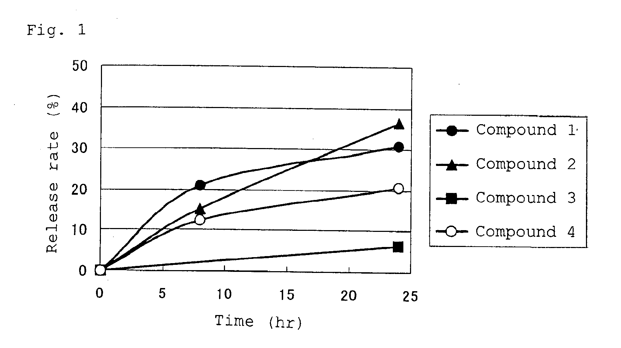

Compound 1, compound 2, compound 3 and compound 4 were respectively dissolved in PBS (phosphate buffered physiological saline: pH 7.1) to a polymer concentration of 1 mg/ml, and the solutions were incubated at 37°C. The resorcinol derivatives released from the high-molecular weight conjugate was separated by HPLC and quantified using a standard curve. The ratio of the quantification value to the total amount of drug determined from the drug content in the high-molecular weight conjugate is presented in Fig. 1.

From the results of the test, the high-molecular weight conjugate of resorcinol compounds of the present invention was confirmed to achieve over tens of hours or more the sustained release of the resorcinol derivatives (the included compound) which exhibit anti-tumor activity, even in the absence of enzymes.

### Test Example 2 Anti-tumor effect on mice transplanted with the mouse colon cancer Colon26

Tumor of mouse colon cancer, Colon26, maintained by serial subcutaneous subculture in BALB/cA mice, was minced into about 2-mm square blocks, and the blocks were transplanted subcutaneously on the dorsal part of a female CDF1 mice with a trocar. On the 7^{th} day after tumor transplantation, the high-molecular weight conjugate of a resorcinol derivative (compound 1) of the present invention, and as a control, the included resorcinol derivative, 5-(2,4-dihydroxy-5-isopropyl-phenyl)-4-(4-methoxy-phenyl)-2,4-dihydro-[1,2,4]triazol-3-one), were administered intravenously to the mouse tail vein. Compound 1 of the present invention was dissolved in a 5% glucose injectable solution, and was administered once. The resorcinol derivative (the included compound) used as the control, was dissolved in DMSO, and TWEEN80 was added thereto, and then the mixture was diluted with a 5% glucose injectable solution. The dilution was administered once a day for 5 consecutive days. After the administration, the major diameter (Lmm) and the minor diameter (W mm) of the tumor were measured regularly, and the tumor volume was calculated by the formula: (L×W²)/2. Based on the tumor volume of the administration initiation day, the average relative tumor volume for each measurement day was determined (Table 1).

**[Table 1]**

| Anti-tumor effect on mice transplanted with mouse colon cancer Colon26 | | | | | | | | | | |
|---|---|---|---|---|---|---|---|---|---|---|
| Name of compound | Administration schedule | Amount of administration | Days after administration | | | | | | | |
| | | mg/kg/day | 0 | 2 | 3 | 5 | 7 | 9 | 12 | 14 |
| Untreated group | - | - | 1.0 | 2.1 | 3.1 | 5.4 | 7.0 | 8.1 | 12 | 14 |
| Compound of the present invention | once | 100 | 1.0 | 0.48 | 0.49 | 0.65 | 0.49 | 0.63 | 0.6 | 0.68 |
| Included compound | Administered for 5 consecutive days | 100 | 1.0 | 1.1 | 0.7 | 1.0 | 3.3 | 5.0 | 11 | 12 |
| | | 50 | 1.0 | 1.6 | 1.6 | 2.5 | 2.5 | 6.7 | 10 | 13 |

As a result, the high-molecular weight conjugate of a resorcinol compound (compound 1) of the present invention suppressed the tumor growth for an extended period of time by single administration, and the anti-tumor effect is enhanced as compared with that obtained by administering the resorcinol derivative (included compound) for 5 consecutive days. That is, it is suggested that the high-molecular weight conjugate of a resorcinol compound has an improved pharmacokinetics and stability in vivo, and maintains the anti-tumor effect over an extended period of time, as compared with the resorcinol derivative. Furthermore, since it is possible to lower the total amount of administration of the included compound, reduced toxicity is also expected. The high-molecular weight conjugate of a resorcinol compound has increased water-solubility compared with the resorcinol derivative, and it is possible to administer the included compound without using DMSO, which is conventionally required for dissolving the included compound.

### Reference Example 1 Synthesis of N-acetylated block copolymer of monomethoxy polyethylene glycol having a molecular weight of about 12,000 and polyglutamic acid having a polymerization number of about 28

Polyethylene glycol having a methoxy group at one end and a 3-aminopropyl group at another end (SUNBRIGHT MEPA-12T, manufactured by Nippon Oil and Fat Co. , Ltd. , average molecular weight 12,000, 1.0 g) was dissolved in DMSO (20 ml), then γ-benzyl L-glutamate N-carboxylic acid anhydride (0.77 g) was added thereto, and the mixture was stirred for 20 hours at 35°C. Ethanol (80 ml) and diisopropyl ether (320 ml) were added to the reaction liquor, and the mixture was stirred for 90 minutes at room temperature. The precipitate was collected by filtration, and washed with ethanol/diisopropyl ether (1/4 (v/v), 100 ml) . The resulting precipitate was dissolved in DMF (20 ml), and acetic anhydride (0.4 ml) was added thereto, and the mixture was stirred for 15 hours at room temperature. Ethanol (80 ml) and diisopropyl ether (320 ml) were added to the reaction liquor, and the mixture was stirred for 90 minutes at room temperature. Then, the precipitate was collected by filtration, and washed with ethanol/diisopropyl ether (1/4 (v/v), 100 ml), to obtain 1.56 g of a polymer. The resulting polymer was dissolved in DMF (47 ml), and then 5% palladium-carbon (780 mg) was added thereto. The mixture was subjected to hydrogenolysis for 3 hours at 35°C. Methanol (90 ml) and Cerite (8 g) were added to the reaction liquor, and stirred for 2 hours, and then 5% palladium-carbon was separated by filtration. Methanol was distilled off under reduced pressure, and then ethanol (90 ml) and diisopropyl ether (360 ml) were added thereto, and stirred for 90 minutes at room temperature. The precipitate was collected by filtration, and washed with ethanol/diisopropyl ether (1/4 (v/v), 100 ml), and the precipitate was dissolved in 10% saline (100 ml). The pH of the solution was adjusted to 10.0 with 1 N aqueous solution of sodium hydroxide, and then the solution was purified using partition adsorption resin column chromatography. The eluted solution was concentrated under reduced pressure, and then freeze-dried to obtain the desired compound (1.18 g). The polymerization number of glutamic acid in one molecule of the compound based on titration using a 0.02 N aqueous solution of sodium hydroxide was about 28. The polymerization number of glutamic acid in one molecule of the compound can be controlled by adjusting the equivalent of the γ-benzyl L-glutamate N-carboxylic acid anhydride.

### Brief Description of the Drawings

Fig. 1 shows the proportion of the amount of released resorcinol derivatives to the total amount of the bound resorcinol derivatives in the PBS solutions (pH 7.1; 37°C) of compound 1 of the present invention (high-molecular weight conjugate in which the included resorcinol derivative is 5-(2,4-dihydroxy-5-isopropyl-phenyl)-4-(4-methoxy-phenyl)-2 ,4-dihydro-[1,2,4]triazol-3-one (formula (5-3))), compound 2 (high-molecular weight conjugate in which the included resorcinol derivative is 4-{5-hydroxy-4-[4-(morpholin-4-yl)-phenyl]-4H-[1,2,4]triazol-3-yl}-6-isopropyl-benzene-1,3-diol (formula (5-8))), compound 3 (high-molecular weight conjugate in which the included resorcinol derivative is 4-(but-2-ynyl)-6-[4-(4-methoxy-phenyl)-5-methyl-isoxazol-3-yl]-benzene-1,3-diol (formula (5-19))), and compound 4 (high-molecular weight conjugate in which the included resorcinol derivative is 5-(2,4-dihydroxy-5-isopropyl-phenyl)-4-isopropyl-2,4-dihydro-[1,2,4]triazol-3-one (formula (5-4))). In Fig. 1, -●- represents the proportion of the released amount of compound 1 of the present invention; -A-, the proportion of compound 2; -■-, the proportion of compound 3; and -0-, the proportion of compound 4.

## Claims

1. A high-molecular weight conjugate of resorcinol derivatives, which is a compound represented by the general formula (1): 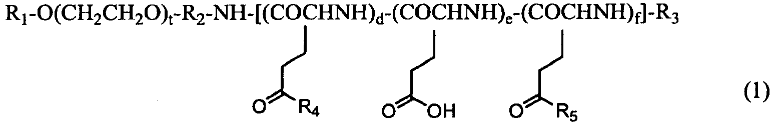 wherein R₁ represents a hydrogen atom, or an alkyl group having 1 to 6 carbon atoms which may have a substituent; R₂ represents a linking group; R₃ represents a hydrogen atom, an acyl group having 1 to 6 carbon atoms, or an alkoxycarbonyl group having 1 to 6 carbon atoms; R₄ represents the residue of the hydroxyl group of the resorcinol derivative; R₅ represents a group selected from the group consisting of an alkoxy group having 1 to 30 carbon atoms, an aralkyloxy group having 7 to 30 carbon atoms, an alkylamino group having 1 to 30 carbon atoms which may have a substituent, a di (C₁-C₃₀) alkylamino group which may have substituent, an amino acid with a protected carboxyl group, and -N(R₆)CONH(R₇) wherein R₆ and R₇, which may be identical or different, each represent a cyclic alkyl group having 3 to 6 carbon atoms, or an alkyl group having 1 to 5 carbon atoms which may be substituted with a tertiary amino group; t represents an integer from 5 to 11500; d represents an integer from 1 to 200, e and f each represent an integer from 0 to 200, and d+e+f represents an integer from 3 to 200; and the respective constituent units of the polyglutamic acid are bound in any order,
wherein the resorcinol derivatives are resorcinol derivatives represented by the general formula (2): 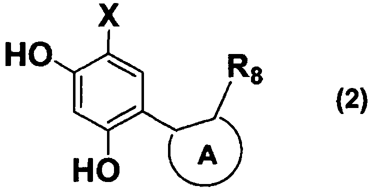 wherein ring A represents a heterocyclic aryl group composed of five atoms, which may have a substituent; X represents a mercapto group, a hydroxyl group, a halogen atom, a nitro group, a cyano group, or an alkyl group, alkenyl group or alkynyl group which may have a substituent, a carbocyclic or heterocyclic aryl group which may have a substituent, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group or carbamoyloxy group, or a group selected from an amino group, an acylamino group, an alkoxycarbonylamino group, a ureido group, a sulfonylamino group, a sulfamoylamino group, a formyl group, an acyl group, a carboxyl group, an alkoxycarbonyl group, a carbamoyl group and a silyl group, which may have a substituent; and R₈ represents a carbocyclic or heterocyclic aryl group which may have a substituent, an alkyl group, alkenyl group or alkynyl group which may have a substituent, or an amino group or acylamino group which may have a substituent, wherein the residues of the resorcinol derivatives are linked by a hydroxyl group to a carboxyl group in the side chain of the copolymer via an ester bond.

2. The high-molecular weight conjugate of resorcinol derivatives according to claim 1, wherein R₁ is an alkyl group having 1 to 6 carbon atoms which may have substituent; R₂ is an alkylene group having 2 to 6 carbon atoms; R₃ is an acyl group having 1 to 6 carbon atoms, or an alkoxycarbonyl group having 1 to 6 carbon atoms; t is an integer from 100 to 300; d is an integer from 1 to 100, e and f are each an integer from 0 to 100, and d+e+f is an integer from 6 to 100; and R₅ is a group selected from the group consisting of an amino acid with a protected carboxyl group and -N(R₆)CONH(R₇).

3. The high-molecular weight conjugate of resorcinol derivatives according to claim 2, wherein R₁ is a methyl group, R₂ is a trimethylene group, R₃ is an acetyl group, and R₅ is an isopropylaminocarbonylisopropylamino group.

4. The high-molecular weight conjugate of resorcinol derivatives according to any one of claims 1 to 3, wherein the ring A defined in claim 1 is any one of the substituents selected from groups of the general formulas (3-1) to (3-7): 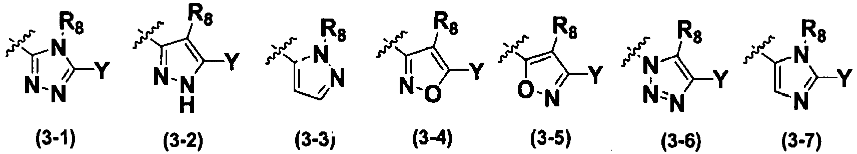 wherein R₈ represents the substituent as defined in claim 1; and Y represents a mercapto group, a hydroxyl group, a hydrogen atom, a halogen atom, a carbamoyl group, an alkoxycarbonyl group, a cyano group, an alkylthio group, an arylthio group, an alkylsulfinyl group, an arylsulfinyl group, an alkylsulfonyl group, an arylsulfonyl group, a sulfamoyl group, an alkoxyl group, an aryloxy group, an acyloxy group, an alkoxycarbonyloxy group, a carbamoyloxy group, or an amino group, an acylamino group, an alkoxycarbonylamino group, a ureido group, a sulfonylamino group, a sulfamoylamino group, a formyl group, an acyl group and a silyl group, which may have a substituent.

5. The high-molecular weight conjugate of resorcinol derivatives according to any one of claims 1 to 4, wherein the resorcinol derivatives are selected from the group consisting of groups of formulas (5-1) to (5-21):

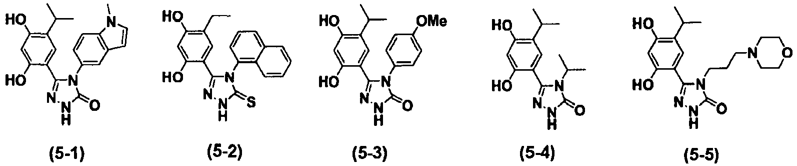

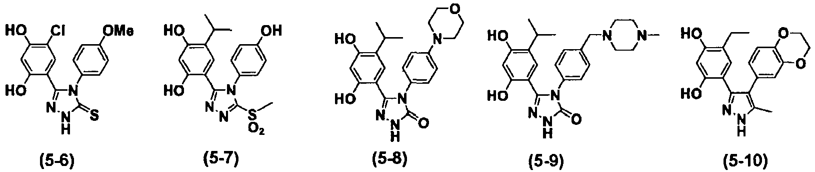

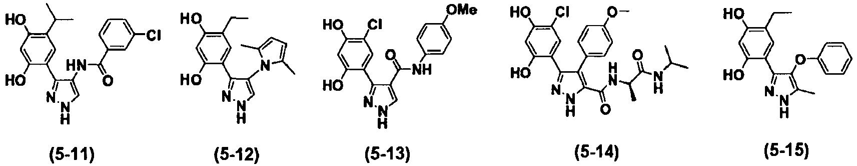

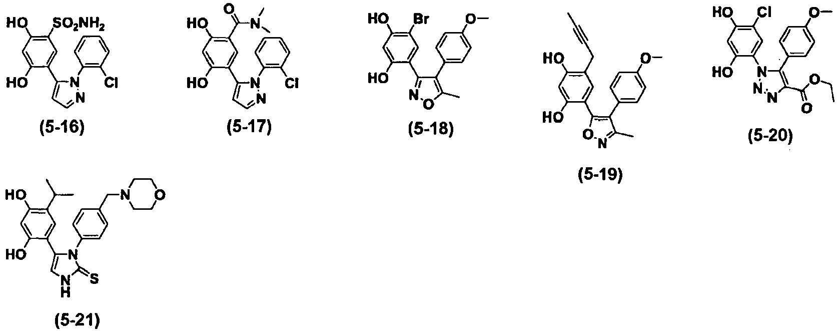

6. A method for producing the high-molecular weight conjugate of resorcinol derivatives according to any one of claims 1 to 5, the method comprising linking the copolymer of a polyethylene glycol moiety and a polymer moiety having a carboxyl group in the side chain to the resorcinol derivatives via an ester bond in an organic solvent, using a dehydrating condensing agent.

7. An anticancer agent comprising the high-molecular weight conjugate of resorcinol derivatives according to any one of claims 1 to 5, as an active ingredient.

## Patentansprüche

1. Resorcinderivatekonjugat mit hohem Molekulargewicht, das eine Verbindung ist, die durch die allgemeine Formel (1) dargestellt ist: 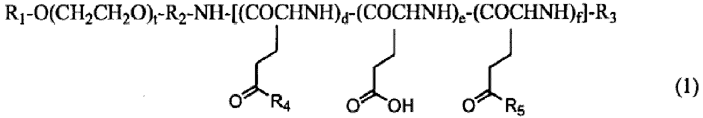 wobei R₁ ein Wasserstoffatom oder eine Alkylgruppe mit 1 bis 6 Kohlenstoffatomen, die einen Substituenten aufweisen können, darstellt; R₂ eine Verknüpfungsgruppe darstellt; R₃ ein Wasserstoffatom, eine Acylgruppe mit 1 bis 6 Kohlenstoffatomen oder eine Alkoxycarbonylgruppe mit 1 bis 6 Kohlenstoffatomen darstellt; R₄ den Rest der Hydroxylgruppe des Resorcinderivats darstellt; R₅ eine Gruppe darstellt, die aus der Gruppe ausgewählt ist, die aus einer Alkoxygruppe mit 1 bis 30 Kohlenstoffatomen, einer Aralkyloxygruppe mit 7 bis 30 Kohlenstoffatomen, einer Alkylaminogruppe mit 1 bis 30 Kohlenstoffatomen, die einen Substituenten aufweisen können, einer Di(C₁-C₃₀)-alkylaminogruppe, die einen Substituenten aufweisen kann, einer Aminosäure mit einer geschützten Carboxylgruppe und -N(R₆)CONH(R₇), wobei R₆ und R₇, die identisch oder unterschiedlich sein können, jeweils eine cyclische Alkylgruppe mit 3 bis 6 Kohlenstoffatomen oder eine Alkylgruppe mit 1 bis 5 Kohlenstoffatomen, die mit einer tertiären Aminogruppe substituiert sein können, besteht; t eine ganze Zahl von 5 bis 11.500 darstellt; d eine ganze Zahl von 1 bis 200 darstellt, e und f jeweils eine ganze Zahl von 0 bis 200 darstellen und d + e + f eine ganze Zahl von 3 bis 200 darstellt; und die jeweiligen Konstitutionseinheiten der Polyglutaminsäure in einer beliebigen Reihenfolge gebunden sind,
wobei die Resorcinderivate Resorcinderivate sind, die durch die allgemeine Formel (2) dargestellt sind: 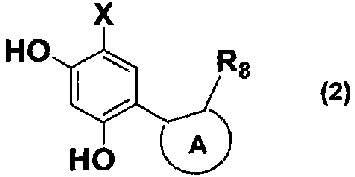 wobei Ring A eine heterocyclische Arylgruppe, bestehend aus fünf Atomen, die einen Substituenten aufweisen können, darstellt; X eine Mercaptogruppe, eine Hydroxylgruppe, ein Halogenatom, eine Nitrogruppe, eine Cyanogruppe oder eine Alkylgruppe, Alkenylgruppe oder Alkinylgruppe, die einen Substituenten aufweisen kann, eine carbocyclische oder heterocyclische Arylgruppe, die einen Substituenten aufweisen kann, eine Alkylthiogruppe, eine Arylthiogruppe, eine Alkylsulfinylgruppe, eine Arylsulfinylgruppe, eine Alkylsulfonylgruppe, eine Arylsulfonylgruppe, eine Sulfamoylgruppe, eine Alkoxygruppe, eine Aryloxygruppe, eine Acyloxygruppe, eine Alkoxycarbonyloxygruppe oder Carbamoyloxygruppe oder eine Gruppe darstellt, die aus einer Aminogruppe, einer Acylaminogruppe, einer Alkoxycarbonylaminogruppe, einer Ureidogruppe, einer Sulfonylaminogruppe, einer Sulfamoylaminogruppe, einer Formylgruppe, einer Acylgruppe, einer Carboxylgruppe, einer Alkoxycarbonylgruppe, einer Carbamoylgruppe und einer Silylgruppe, die einen Substituenten aufweisen können, ausgewählt ist; und R₈ eine carbocyclische oder heterocyclische Arylgruppe, die einen Substituenten aufweisen kann, eine Alkylgruppe, Alkenylgruppe oder Alkinylgruppe, die einen Substituenten aufweisen kann, oder eine Aminogruppe oder Acylaminogruppe, die einen Substituenten aufweisen kann, darstellt, wobei die Reste der Resorcinderivate durch eine Hydroxylgruppe mit einer Carboxylgruppe in der Seitenkette des Copolymers über eine Esterbindung verknüpft sind.

2. Resorcinderivatekonjugat mit hohem Molekulargewicht nach Anspruch 1, wobei R₁ eine Alkylgruppe mit 1 bis 6 Kohlenstoffatomen ist, die einen Substituenten aufweisen können; R₂ eine Alkylengruppe mit 2 bis 6 Kohlenstoffatomen ist; R₃ eine Acylgruppe mit 1 bis 6 Kohlenstoffatomen oder eine Alkoxycarbonylgruppe mit 1 bis 6 Kohlenstoffatomen ist; t eine ganze Zahl von 100 bis 300 ist; d eine ganze Zahl von 1 bis 100 ist, e und f jeweils eine ganze Zahl von 0 bis 100 sind und d + e + f eine ganze Zahl von 6 bis 100 ist; und R₅ eine Gruppe ist, die aus der Gruppe ausgewählt ist, die aus einer Aminosäure mit einer geschützten Carboxylgruppe und -N(R₆)CONH(R₇) besteht.

3. Resorcinderivatekonjugat mit hohem Molekulargewicht nach Anspruch 2, wobei R₁ eine Methylgruppe ist, R₂ eine Trimethylengruppe ist, R₃ eine Acetylgruppe ist und R₅ eine Isopropylaminocarbonylisopropylamino-Gruppe ist.

4. Resorcinderivatekonjugat mit hohem Molekulargewicht nach einem von Anspruch 1 bis 3, wobei der Ring A, der in Anspruch 1 definiert ist, ein beliebiger der Substituenten ist, die aus Gruppen mit der allgemeinen Formel (3-1) bis (3-7) ausgewählt sind: 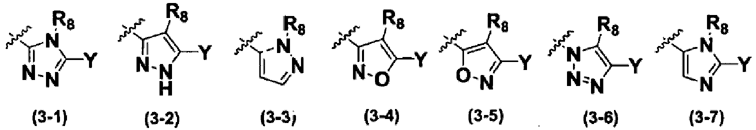 wobei R₈ den Substituenten, wie in Anspruch 1 definiert, darstellt; und Y eine Mercaptogruppe, eine Hydroxylgruppe, ein Wasserstoffatom, ein Halogenatom, eine Carbamoylgruppe, eine Alkoxycarbonylgruppe, eine Cyanogruppe, eine Alkylthiogruppe, eine Arylthiogruppe, eine Alkylsulfinylgruppe, eine Arylsulfinylgruppe, eine Alkylsulfonylgruppe, eine Arylsulfonylgruppe, eine Sulfamoylgruppe, eine Alkoxygruppe, eine Aryloxygruppe, eine Acyloxygruppe, eine Alkoxycarbonyloxygruppe, eine Carbamoyloxygruppe oder eine Aminogruppe, eine Acylaminogruppe, eine Alkoxycarbonylaminogruppe, eine Ureidogruppe, eine Sulfonylaminogruppe, eine Sulfamoylaminogruppe, eine Formylgruppe, eine Acylgruppe und eine Silylgruppe, die einen Substituenten aufweisen können, darstellt.

5. Resorcinderivatekonjugat mit hohem Molekulargewicht nach einem von Anspruch 1 bis 4, wobei die Resorcinderivate aus der Gruppe ausgewählt sind, die aus Gruppen mit der Formel (5-1) bis (5-21) besteht:

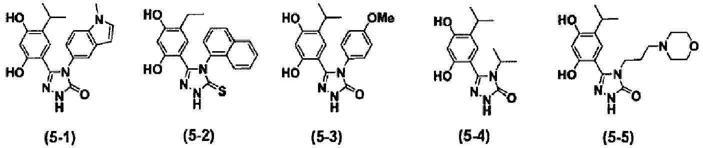

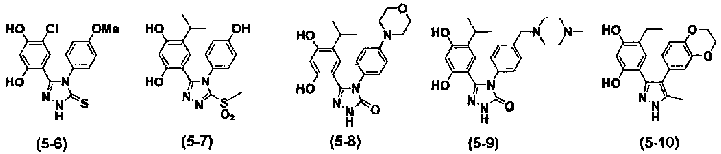

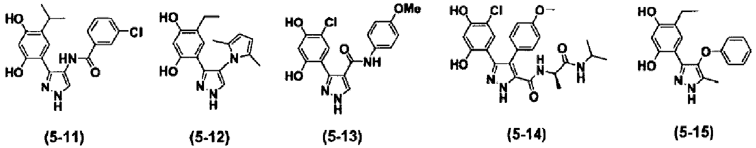

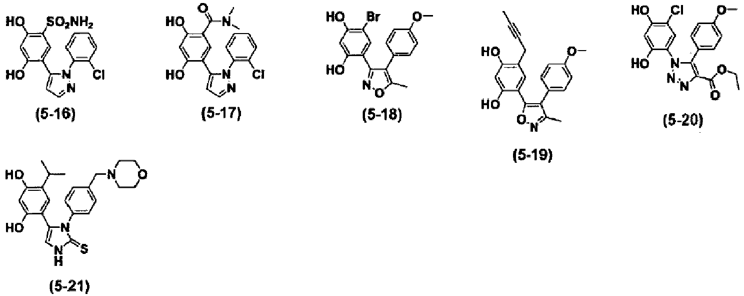

6. Verfahren zum Herstellen des Resorcinderivatekonjugats mit hohem Molekulargewicht nach einem von Anspruch 1 bis 5, wobei das Verfahren das Verknüpfen des Copolymers aus einem Polyethylenglycol-Molekülteil und einem Polymer-Molekülteil, der eine Carboxylgruppe in der Seitenkette aufweist, mit den Resorcinderivaten über eine Esterbindung in einem organischen Lösemittel unter Benutzen eines wasserentziehenden Kondensationsmittels umfasst.

7. Antikrebsmedikament, umfassend das Resorcinderivatekonjugat mit hohem Molekulargewicht nach einem von Anspruch 1 bis 5 als einen aktiven Inhaltsstoff.

## Revendications

1. Conjugué de dérivés de résorcinol de masse moléculaire élevée, qui est un composé représenté par la formule générale (1) : 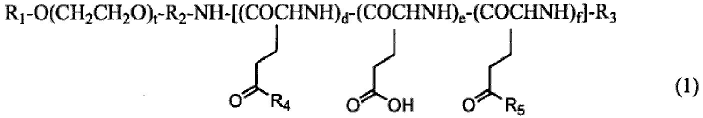 dans laquelle R₁ représente un atome d'hydrogène, ou un groupe alkyle ayant de 1 à 6 atomes de carbone qui peuvent avoir un substituant ; R₂ représente un groupe de liaison ; R₃ représente un atome d'hydrogène, un groupe acyle ayant de 1 à 6 atomes de carbone, ou un groupe alcoxycarbonyle ayant de 1 à 6 atomes de carbone ; R₄ représente le résidu du groupe hydroxyle du dérivé de résorcinol ; R₅ représente un groupe sélectionné dans le groupe constitué d'un groupe alcoxy ayant de 1 à 30 atomes de carbone, un groupe aralkyloxy ayant de 7 à 30 atomes de carbone, un groupe alkylamino ayant de 1 à 30 atomes de carbone qui peuvent avoir un substituant, un groupe di(alkyl en C₁-C₃₀)amino qui peut avoir un substituant, un acide aminé avec un groupe carboxyle protégé, et - N(R₆)CONH(R₇) dans laquelle R₆ et R₇, qui peuvent être identiques ou différents, représentent chacun un groupe alkyle cyclique ayant de 3 à 6 atomes de carbone, ou un groupe alkyle ayant de 1 à 5 atomes de carbone qui peut être substitué par un groupe amino tertiaire ; t représente un nombre entier de 5 à 11 500 ; d représente un nombre entier de 1 à 200, e et f représentent chacun un nombre entier de 0 à 200, et d + e + f représentent un nombre entier de 3 à 200 ; et les unités constitutives respectives de l'acide polyglutamique sont liées dans tout ordre,
dans lequel les dérivés du résorcinol sont représentés par la formule générale (2) : 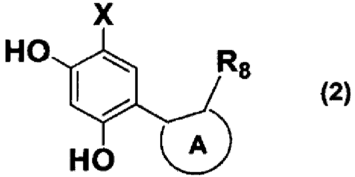 dans laquelle le cycle A représente un groupe aryle hétérocyclique composé de cinq atomes, pouvant avoir un substituant ; X représente un groupe mercapto, un groupe hydroxyle, un atome d'halogène, un groupe nitro, un groupe cyano, ou un groupe alkyle, un groupe alcényle ou un groupe alcynyle qui peuvent avoir un substituant, un groupe aryle carbocyclique ou hétérocyclique qui peut avoir un substituant, un groupe alkylthio, un groupe arylthio, un groupe alkylsulfinyle, un groupe arylsulfinyle, un groupe alkylsulfonyle, un groupe arylsulfonyle, un groupe sulfamoyle, un groupe alcoxy, un groupe aryloxy, un groupe acyloxy, un groupe alcoxycarbonyloxy ou un groupe carbamoyloxy, ou un groupe sélectionné parmi un groupe amino, un groupe acylamino, un groupe alcoxycarbonylamino, un groupe uréido, un groupe sulfonylamino, un groupe sulfamoylamino, un groupe formyle, un groupe acyle, un groupe carboxyle, un groupe alcoxycarbonyle, un groupe carbamoyle et un groupe silyle, qui peuvent avoir un substituant ; et R₈ représente un groupe aryle carbocyclique ou hétérocyclique qui peut avoir un substituant, un groupe alkyle, un groupe alcényle ou un groupe alcynyle qui peuvent avoir un substituant, ou un groupe amino ou un groupe acylamino qui peuvent avoir un substituant, dans lequel les résidus des dérivés de résorcinol sont liés par un groupe hydroxyle à un groupe carboxyle dans la chaîne latérale du copolymère via une liaison ester.

2. Conjugué de dérivés de résorcinol de masse moléculaire élevée selon la revendication 1, dans lequel R₁ est un groupe alkyle ayant de 1 à 6 atomes de carbone qui peut avoir un substituant ; R₂ est un groupe alkylène ayant de 2 à 6 atomes de carbone ; R₃ est un groupe acyle ayant de 1 à 6 atomes de carbone, ou un groupe alcoxycarbonyle ayant de 1 à 6 atomes de carbone ; t est un nombre entier de 100 à 300 ; d est un nombre entier de 1 à 100, e et f sont chacun un nombre entier de 0 à 100, et d + e + f est un nombre entier de 6 à 100 ; et R₅ est un groupe sélectionné dans le groupe constitué d'un acide aminé avec un groupe carboxyle protégé et -N(R₆)CONH(R₇).

3. Conjugué de dérivés de résorcinol de masse moléculaire élevée selon la revendication 2, dans lequel R₁ est un groupe méthyle, R₂ est un groupe triméthylène, R₃ est un groupe acétyle, et R₅ est un groupe isopropylaminocarbonylisopropylamino.

4. Conjugué de dérivés de résorcinol de masse moléculaire élevée selon l'une quelconque des revendications 1 à 3, dans lequel le cycle A défini dans la revendication 1 est l'un quelconque des substituants sélectionnés dans les groupes de formules générales (3-1) à (3-7) : 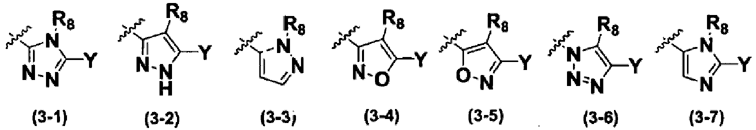 dans lesquelles R₈ représente le substituant tel que défini dans la revendication 1 ; et Y représente un groupe mercapto, un groupe hydroxyle, un atome d'hydrogène, un atome d'halogène, un groupe carbamoyle, un groupe alcoxycarbonyle, un groupe cyano, un groupe alkylthio, un groupe arylthio, un groupe alkylsulfinyle, un groupe arylsulfinyle, un groupe alkylsulfonyle, un groupe arylsulfonyle, un groupe sulfamoyle, un groupe alcoxyle, un groupe aryloxy, un groupe acyloxy, un groupe alcoxycarbonyloxy, un groupe carbamoyloxy, ou un groupe amino, un groupe acylamino, un groupe alcoxycarbonylamino, un groupe uréido, un groupe sulfonylamino, un groupe sulfamoylamino, un groupe formyle, un groupe acyle et un groupe silyle, qui peuvent avoir un substituant.

5. Conjugué de dérivés de résorcinol de masse moléculaire élevée selon l'une quelconque des revendications 1 à 4, dans lequel les dérivés de résorcinol sont sélectionnés dans le groupe constitué des groupes de formules (5-1) à (5-21) :

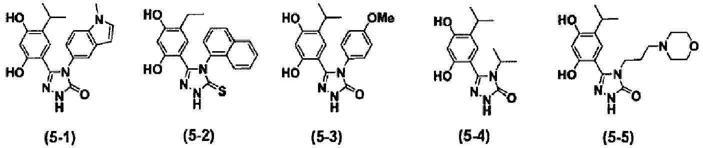

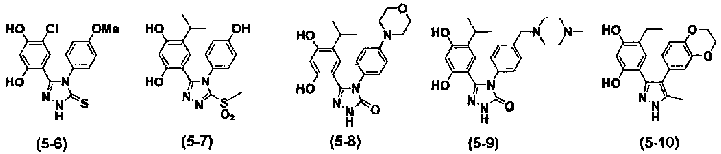

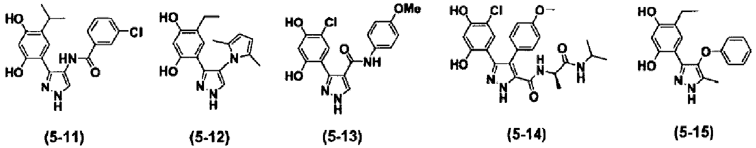

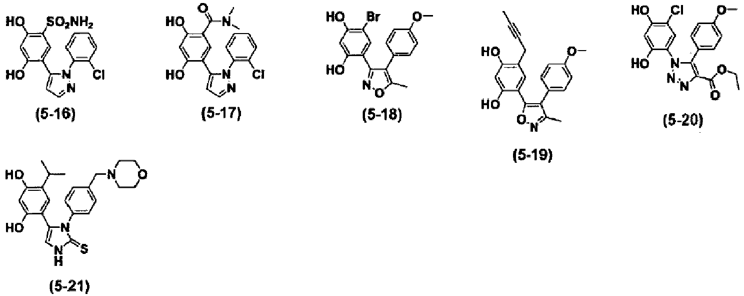

6. Procédé de production du conjugué de dérivés de résorcinol de masse moléculaire élevée selon l'une quelconque des revendications 1 à 5, le procédé comprenant la liaison du copolymère d'une fraction polyéthylène glycol et d'une fraction polymère ayant un groupe carboxyle dans la chaîne latérale aux dérivés de résorcinol via une liaison ester dans un solvant organique, en utilisant un agent de condensation déshydratant.

7. Agent anticancéreux comprenant le conjugué de dérivés de résorcinol de masse moléculaire élevée selon l'une quelconque des revendications 1 à 5, en tant que principe actif.
